# EUROPEAN PATENT SPECIFICATION

(11) **EP 4 111 955 B1**
(45) Date of publication and mention of the grant of the patent: **08.10.2025**
(21) Application number: 21759740.0
(22) Date of filing: 24.02.2021
(51) Int. Cl.: A61B 5/029, A61B 5/028, A61B 5/021, A61B 5/00, G16H 50/20, G06N 3/08, G06N 3/04

(54) **DEVICE AND METHOD FOR CALCULATING STROKE VOLUME USING AI**
VORRICHTUNG UND VERFAHREN ZUR BERECHNUNG DES SCHLAGVOLUMENS MIT KÜNSTLICHER INTELLIGENZ
DISPOSITIF ET PROCÉDÉ DE CALCUL DE VOLUME SYSTOLIQUE EN UTILISANT L'IA

(30) Priority: 25.02.2020 KR 20200022914
(43) Date of publication of application: 04.01.2023
(73) Proprietor: Seoul National University Hospital, Seoul 03080 (KR); Daegu Gyeongbuk Institute Of Science and Technology, Daegu 42988 (KR)
(72) Inventor: JUNG, Chul Woo, Seoul 03080 (KR); LEE, Hyung Chul, Seoul 03080 (KR); YANG, Hyun Lim, Dalseong-gun Daegu 42988 (KR); KIM, Min Soo, Dalseong-gun Daegu 42988 (KR)
(74) Representative: dompatent von Kreisler Selting Werner - Partnerschaft von Patent- und Rechtsanwälten mbB
(86) International application number: PCT/KR2021/002274
(87) International publication number: WO 2021/172852

(56) References cited:
- JP-A- 2008 506 472
- JP-A- 2010 246 801
- KR-A- 20130 095 862
- KR-A- 20130 095 862
- KR-A- 20170 073 051
- KR-A- 20200 006 447
- KR-A- 20200 006 447
- BENJAMIN PRATT ET AL: "Calculating Arterial Pressure-Based Cardiac Output Using a Novel Measurement and Analysis Method", 1 January 2007 (2007-01-01), online, pages 403 - 411, XP055086642, Retrieved from the Internet <URL:http://www.researchgate.net/publication/5853603_Calculating_arterial_pressure-based_cardiac_output_using_a_novel_measurement_and_analysis_method/file/79e415097ff64b8867.pdf> [retrieved on 20131104], DOI: 10.2345/0899-8205(2007)41[403:CAPCOU]2.0.CO;2
- BIGHAMIAN RAMIN ET AL: "Relationship between Stroke Volume and Pulse Pressure during Blood Volume Perturbation: A Mathematical Analysis", BIOMED RESEARCH INTERNATIONAL, vol. 2014, 20 May 2014 (2014-05-20), pages 1 - 10, XP093125860, ISSN: 2314-6133, Retrieved from the Internet <URL:https://www.ncbi.nlm.nih.gov/pmc/articles/PMC4054969/pdf/BMRI2014-459269.pdf> DOI: 10.1155/2014/459269

## Description

### [Technical Field]

The present invention relates to a device and method for calculating stroke volume using artificial intelligence (AI), which calculate a stroke volume (i.e., cardiac output) using a stroke volume calculation model generated by AI to accurately calculate the stroke volume from an arterial blood pressure.

### [Background Art]

Conventionally, as a method for calculating cardiac stroke volume in a patient, there are a method for calculating stroke volume using arterial pressure-based cardiac output (APCO) equipment which calculates an arterial blood pressure (ABP) waveform-based stroke volume, and a method for calculating stroke volume using thermodilution-based cardiac output (TDCO) equipment which calculates a stroke volume by directly inserting a catheter into a patient (A thermodilution method: a method for determining a flow rate by injecting a fluid B (as a temperature indicator) having a known temperature into a certain fluid A at a constant rate, and measuring a temperature change at downstream therefrom. That is, a method for calculating a blood flow rate by measuring the temperature change by the injected indicator B, which is determined by the fluid A passing through the injected portion).

The conventional method for calculating stroke volume using the APCO equipment is less invasive than the TDCO equipment which directly inserts the catheter into patient's body, but it is inaccurate compared to the TDCO equipment since the stroke volume is mathematically calculated using an average or standard deviation of pulse pressures per beat. In addition, the method for calculating stroke volume using the APCO equipment tends not to predict a high stroke volume.

In addition, the method for calculating stroke volume using the TDCO equipment, which is the current gold standard (a test to diagnose the progress of disease), is more accurate than the method for calculating stroke volume using the APCO equipment, but it is more invasive and has a problem that causes complications such as infection, etc.

Relevant prior art is disclosed in
KR 2020 0006447 A;
KR 2013 0095862 A;
Benjamin Pratt ET AL: "Calculating Arterial Pressure-Based Cardiac Output Using a Novel Measurement and Analysis Method",, 1 January 2007 (2007-01-01), pages 403-411;
Bighamian Ramin ET AL: "Relationship between Stroke Volume and Pulse Pressure during Blood Volume Perturbation: A Mathematical Analysis",BioMed Research International, vol. 2014, 20 May 2014 (2014-05-20), pages 1-10

### [Summary of Invention]

### [Problems to be Solved by Invention]

In consideration of the above-mentioned circumstances, it is an object of the present invention to implement a device and method for calculating stroke volume with high accuracy while being less invasive to a patient than conventional ones.

### [Means for Solving Problems]

To achieve the above object, according to an aspect of the present invention, there is provided a device for calculating stroke volume using AI comprising:
a filtering unit configured to filter an arterial blood pressure value and a stroke volume which are in a preset range, among first data and second data comprising a plurality of arterial blood pressure values and a plurality of stroke volumes corresponding to the plurality of arterial blood pressure values;
a pre-training unit configured to pre-train a first stroke volume calculation model which calculates a stroke volume based on the arterial blood pressure value, by using third data filtered from the first data;
a transfer learning unit configured to transfer learn the first stroke volume calculation model, by using fourth data filtered from the second data, thus to generate a second stroke volume calculation model; and
a stroke volume calculation unit configured to calculate a stroke volume corresponding to the input arterial blood pressure of a specific patient by using the second stroke volume calculation model, wherein the filtering unit comprises:
   a data extraction unit configured to extract arterial blood pressure values and corresponding stroke volumes from a preset time before a first time point to the first time point, from the first data and the second data, wherein the first time point is a time point where the change slope of the stroke volume waveform reaches or exceeds a preset slope value.

According to another aspect of the present invention, there is provided a method for calculating stroke volume using AI which calculates a stroke volume from an arterial blood pressure of a specific patient by using a device for calculating stroke volume using AI, the method comprising:
filtering an arterial blood pressure value and a stroke volume which are in a preset range, among first data and second data comprising a plurality of arterial blood pressure values and a plurality of stroke volumes corresponding to the plurality of arterial blood pressure values;
pre-training a first stroke volume calculation model which calculates a stroke volume based on the arterial blood pressure value, by using third data filtered from the first data;transfer-learning the first stroke volume calculation model, by using fourth data filtered from the second data, thus to generate a second stroke volume calculation model; and
calculating astroke volume corresponding to the input arterial blood pressure of a specific patient by using the second stroke volume calculation model, wherein the filtering comprising:
   extracting arterial blood pressure values and corresponding stroke volumes from a preset time before a preset time point to the first time point, from the first data and the second data,
   wherein the first time point is a time point where the change slope of the stroke volume waveform reaches or exceeds a preset slope value.

### [Advantageous effects]

According to embodiments of the present invention, it is possible to predict a stroke volume without purchasing an expensive dedicated catheter by calculating a more accurate stroke volume using blood pressure waveform data output from a commercial patient monitor.

According to embodiments of the present invention, the catheter is not directly injected into patient's central vein or pulmonary artery, such that an accurate stroke volume may be easily calculated in a less invasive manner.

### [Brief Description of Drawings]

FIG. 1 is a block diagram schematically illustrating a configuration of a system for calculating stroke volume using AI according to an embodiment of the present invention.
FIG. 2A is a block diagram schematically illustrating a device for calculating stroke volume using AI according to an embodiment of the present invention.
FIG. 2B is a block diagram schematically illustrating a filtering unit of the device for calculating stroke volume using AI according to an embodiment of the present invention.
FIG. 2C is a block diagram schematically illustrating a model training unit of the device for calculating stroke volume using AI according to an embodiment of the present invention.
FIG. 3 is a graph illustrating an arterial blood pressure in a specific patient and a stroke volume calculated therefrom.
FIG. 4 is a flowchart illustrating a sequence of a method for calculating stroke volume using AI according to an embodiment of the present invention.
FIG. 5 is a flowchart illustrating a sequence of the method for calculating stroke volume using AI according to an embodiment of the present invention.
FIG. 6 is a flowchart illustrating a sequence of the method for calculating stroke volume using AI according to an embodiment of the present invention.
FIG. 7 is a diagram illustrating an example of a sequence in which a stroke volume is calculated using a stroke volume calculation model according to an embodiment of the present invention.

### [Mode for Carrying out Invention]

Hereinafter, specific embodiments of the present invention will be described with reference to the accompanying drawings. The following detailed description is provided to contribute to a comprehensive understanding of a method, apparatus, and/or system described herein. However, these embodiments merely illustrative examples, and the present invention is not limited thereto.

In descriptions of the embodiments of the present invention, publicly known techniques that are judged to be able to make the purport of the present invention unnecessarily obscure will not be described in detail. In addition, the terms as used herein are defined by taking functions of the present disclosure into account and can be changed according to the custom or intention of users or operators. Therefore, definition of the terms should be made according to the overall disclosure set forth herein. In addition, the terminology used herein is for the purpose of describing particular embodiments only and is not intended to limit the present invention thereto. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, and a part or a combination thereof, but do not preclude the presence or addition of one or more other features, integers, steps, operations, elements, components, and a part or a combination thereof.

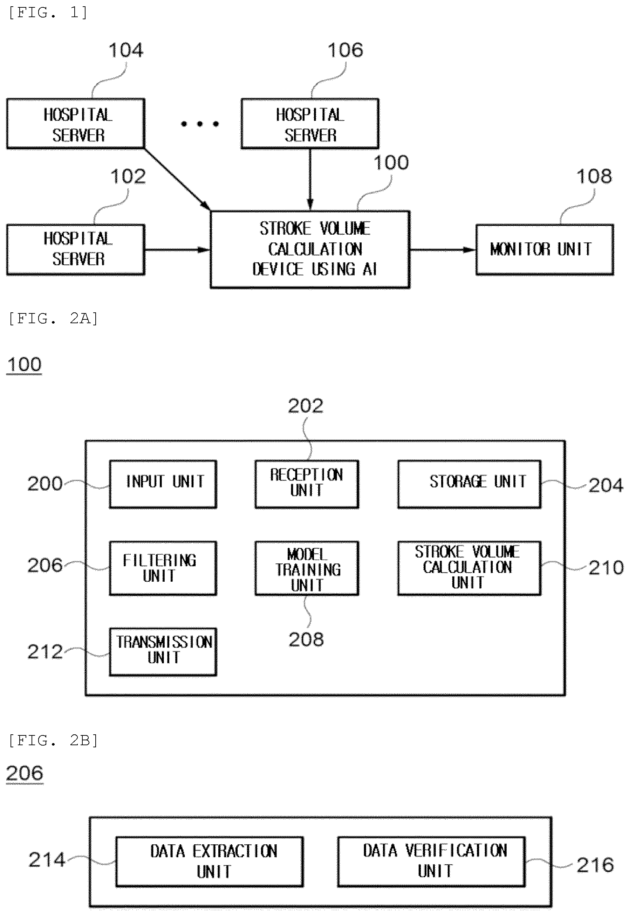

FIG. 1 is a block diagram schematically illustrating a configuration of a system for calculating stroke volume using AI according to an embodiment of the present invention.

The system for calculating stroke volume using AI includes a plurality of hospital servers (or servers of each doctor) 102, 104 and 106, a monitor unit 108 for displaying a stroke volume (or cardiac output) waveform, and a stroke volume calculation device 100 using AI.

The stroke volume calculation device 100 using AI receives arterial blood pressures of a plurality of patients and stroke volume information calculated based on the arterial blood pressures, which are respectively stored in the servers, from the plurality of hospital servers 102, 104 and 106. The stroke volumes calculated based on the arterial blood pressures of the plurality of patients include stroke volume information calculated using APCO equipment and stroke volume information calculated using TDCO equipment. However, the stroke volume calculation device 100 using AI may receive 100 Hz arterial blood pressure waveform and stroke volume data from a public data set, for example, Vital DB instead of the plurality of hospital servers.

The stroke volume calculated based on the arterial blood pressure measured using the APCO equipment and arterial blood pressure information corresponding thereto are referred to as first data. The stroke volume data calculated using the TDCO equipment is referred to as second data.

In addition, the stroke volume calculation device 100 using AI receives patient's information corresponding to the first data and the second data from the plurality of hospital servers 102, 104 and 106 or the public data set, respectively. The patient's information may include a gender, age, weight, height, etc. of the patient. Further, the patient's information may further include information related to body or health condition of the patient.

The stroke volume calculation device 100 using AI stores the received first data, second data, and patient's information respectively corresponding to the first data and the second data.

The stroke volume calculation device 100 using AI uses the first data and the second data for training a stroke volume calculation model generated based on these data. The stroke volume calculation device 100 using AI extracts sample data based on the stored first data and the second data for use in the stroke volume calculation model.

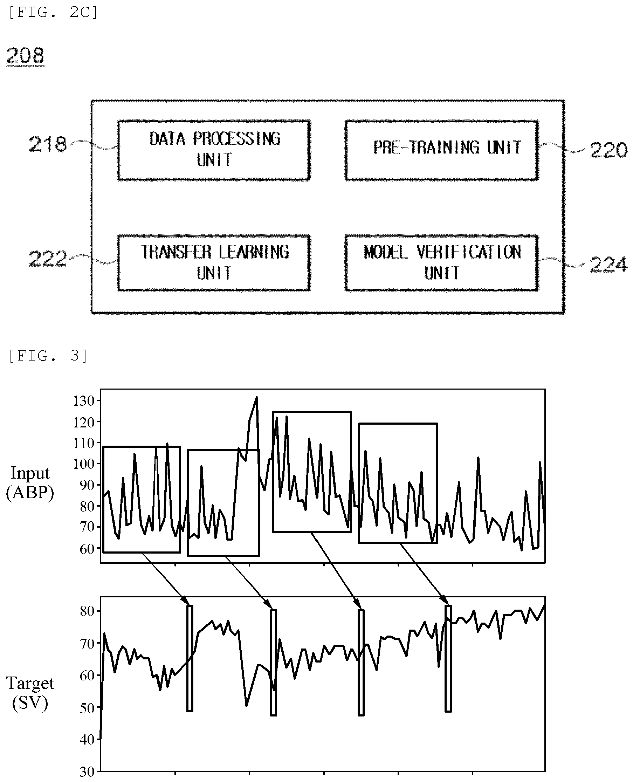

Specifically, as seen from the graph shown in FIG. 3, the stroke volume calculation device 100 using AI observes stroke volume waveforms in the first data and the second data, thus to extract arterial blood pressure values from before a preset time to a first time point at the first time point where a change slope at which the stroke volume waveform changes at an arbitrary point in time is a preset slope value or more. In addition, the device extracts stroke volumes corresponding to the extracted arterial blood pressure values. The preset time may be, for example, 20 seconds.

The stroke volume calculation device 100 using AI performs data verification on the arterial blood pressure values extracted from the first data and the second data, respectively, and the stroke volumes corresponding to the extracted arterial blood pressure values. The stroke volume calculation device 100 using AI verifies the extracted sample data based on a predetermined reference. The stroke volume calculation device 100 using AI determines whether the arterial blood pressure value among the sample data extracted from the first data or the sample data extracted from the second data is within a first preset range. The stroke volume calculation device 100 using AI does not use a value in which the arterial blood pressure value among the extracted sample data is not within the first preset range in training of the stroke volume calculation model. The first preset range may be, for example, 25 or more and 250 or less. That is, when the arterial blood pressure value among the extracted sample data is less than 25 or more than 250, the stroke volume calculation device 100 using AI excludes it from the sample data. The case in which the arterial blood pressure value is less than 25 or more than 250 corresponds to a range where clinically appearing cases are very rare. By limiting the arterial blood pressure value to 25 or more and 250 or less, it is possible to prevent data being recorded in a state in which the catheter is not inserted into the patient's artery or in which there are artifacts due to flushing of an arterial canal from being used as training data.

In addition, the stroke volume calculation device 100 using AI determines whether the stroke volume among the sample data extracted from the first data or the sample data extracted from the second data is within a second preset range. The stroke volume calculation device 100 using AI does not use a value in which the stroke volume among the extracted sample data is not within the second preset range in training of the stroke volume calculation model. The second preset range may be, for example, 20 or more and 200 or less. That is, when the stroke volume among the extracted sample data is less than 20 or more than 200, the stroke volume calculation device 100 using AI excludes it from the sample data. The case in which the stroke volume is less than 20 or more than 200 corresponds to a range where clinically appearing cases are very rare. By limiting the stroke volume to 20 or more and 200 or less, it is possible to prevent a malfunction result of the stroke volume calculation model that may occur from being provided.

In addition, the stroke volume calculation device 100 using AI also excludes data from the sample data, in which a standard deviation per bit calculated from an arterial blood pressure waveform is zero (0), among the sample data extracted from the first data or sample data extracted from the second data.

The data verified by the stroke volume calculation device 100 using AI as described above are referred to as third data or fourth data, respectively. That is, data verified after data extraction among the first data is referred to as the third data, and data verified after data extraction among the second data is referred to as the fourth data.

The stroke volume calculation device 100 using AI performs data processing in order to use the third data and the fourth data in training of the stroke volume calculation model, respectively.

The stroke volume calculation device 100 using AI may smooth the stroke volume value through a method of performing preset smoothing on the stroke volume among the third data. The preset smoothing method may be, for example, Lowess smoothing. This is because the stroke volume calculated by the APCO equipment is calculated by an equation and is output discretely. Therefore, this operation may be performed so as to match the output stroke volume with the stroke volume calculated based on the TDCO equipment, which is output as continuous and smoothed data.

In addition, the stroke volume calculation device 100 using AI may perform delay processing on the fourth data as much as a preset time period, for example, 2 minutes. This is because the TDCO equipment outputs data with a slight delay when actually outputting the same. Therefore, this operation is intended to use the value subjected to delay averaging ("delay-averaged value") in training so as to reflect it in the actual modeling. However, the stroke volume calculation device 100 using AI may use the fourth data as it is without performing the delay processing.

The stroke volume calculation device 100 using AI trains the stroke volume calculation model, which is a deep learning model, by using the third data subjected to smoothing processing ("smooth-processed third data") and patient's information matching with the third data (first data). The stroke volume calculation model calculates a stroke volume for a specific patient when inputting information such as an arterial blood pressure, gender, age, height, weight, etc. of the specific patient.

Next, the stroke volume calculation device 100 using AI transfer learns the stroke volume calculation model subjected to training ("trained stroke volume calculation model"), by using the fourth data subjected to delay averaging ("delay-averaged fourth data") and information of the specific patient matching with the fourth data (second data). However, the fourth data which is not subjected to delay averaging may be used here. Finally, the device verifies accuracy of the stroke volume calculation model measured with a pulmonary artery catheter collected prospectively using the stroke volume calculation model subjected to transfer learning ("transfer-learned stroke volume calculation model").

When using the finally verified stroke volume calculation model, the stroke volume calculation device 100 using AI calculates the stroke volume as a more accurate value using the arterial blood pressure of the specific patient measured by existing commercial equipment.

FIG. 2A is a block diagram schematically illustrating a device for calculating stroke volume using AI according to an embodiment of the present invention.

The stroke volume calculation device 100 using AI includes an input unit 200, a reception unit 202, a storage unit 204, a filtering unit 206, a model training unit 208, a stroke volume calculation unit 210, and a transmission unit 212.

However, the stroke volume calculation device 100 using AI does not include the stroke volume calculation unit 210 and may transmit the trained stroke volume calculation model to an external device.

The input unit 200 may be configured to input information of a specific patient, for example, information on the age, gender, height, and weight of the specific patient. In addition, the input unit 200 may be configured to input the stroke volumes for a plurality of patients and arterial blood pressure information corresponding thereto, which are calculated through the APCO equipment. In addition, the input unit 200 may be configured to input the stroke volumes for a plurality of patients and arterial blood pressure information corresponding thereto, which are calculated through the TDCO equipment.

The reception unit 202 receives the arterial blood pressures of the plurality of patients and the stroke volume information calculated based on the arterial blood pressures, which are respectively stored in the servers, from the plurality of hospital servers 102, 104 and 106. The stroke volumes calculated based on the arterial blood pressures of the plurality of patients include stroke volume information calculated using the APCO equipment and stroke volume information calculated using the TDCO equipment. However, the reception unit 202 may receive 100 Hz arterial blood pressure waveform and stroke volume data from a public data set, for example, Vital DB instead of the plurality of hospital servers.

The stroke volume calculated based on the arterial blood pressure measured using the APCO equipment and arterial blood pressure information corresponding thereto are referred to as first data. The stroke volume data calculated using the TDCO equipment is referred to as second data.

The reception unit 202 receives patient's information respectively corresponding to the first data and the second data from the plurality of hospital servers 102, 104 and 106 or the public dataset. The patient's information may include the gender, age, weight, height, etc. of the patient. In addition, the patient's information may further include information related to the body or health condition of the patient.

The storage unit 204 stores the arterial blood pressure information for the specific patient input from the input unit 200, and the stroke volume information corresponding thereto by matching with information such as the age, gender, height, and weight thereof. In addition, the storage unit 204 stores the stroke volume calculated based on the arterial blood pressures of the plurality of patients received from the reception unit 202 and the arterial blood pressure information corresponding thereto by matching with each patient's information (e.g., the gender, age, weight, height, etc. of the patient).

Further, the storage unit 204 also stores the third data and the fourth data extracted and verified by the filtering unit 206, respectively. In addition, the storage unit 204 stores an algorithm of the stroke volume calculation model subjected to training in the model training unit 208.

The filtering unit 206 extracts and verifies specific data among the first data and the second data to generate third data and fourth data to be used in the model training unit 208.

The filtering unit 206 observes stroke volume waveforms in the first data and the second data, thus to extract arterial blood pressure values from before a preset time to a first time point at the first time point where a change slope at which the stroke volume waveform changes at an arbitrary point in time is a preset slope value or more. In addition, the unit extracts stroke volumes corresponding to the extracted arterial blood pressure values. The preset time may be, for example, 20 seconds.

Next, the filtering unit 206 performs data verification on the arterial blood pressure values extracted from the first data and the second data, respectively, and the stroke volumes corresponding to the extracted arterial blood pressure values. The filtering unit 206 determines whether the arterial blood pressure value among the sample data extracted from the first data or the sample data extracted from the second data is within a first preset range. The filtering unit 206 does not use a value in which the arterial blood pressure value among the extracted sample data is not within the first preset range in training of the stroke volume calculation model. The first preset range may be, for example, 25 or more and 250 or less. That is, when the arterial blood pressure value among the extracted sample data is less than 25 or more than 250, the filtering unit 206 excludes it from the sample data.

In addition, the filtering unit 206 determines whether the stroke volume among the sample data extracted from the first data or the sample data extracted from the second data is within a second preset range. The filtering unit 206 does not use a value in which the stroke volume among the extracted sample data is not within the second preset range in training of the stroke volume calculation model. The second preset range may be, for example, 20 or more and 200 or less. That is, when the stroke volume among the extracted sample data is less than 20 or more than 200, the filtering unit 206 excludes it from the sample data.

In addition, the filtering unit 206 also excludes data from the sample data, in which the calculated standard deviation per bit is zero (0), among the sample data extracted from the first data or sample data extracted from the second data.

The data verified by the filtering unit 206 as described above are referred to as third data or fourth data, respectively. That is, data verified after data extraction among the first data is referred to as third data, and data verified after data extraction among the second data is referred to as fourth data. The third data and the fourth data finally extracted and verified by the filtering unit 206 are stored in the storage unit 204 by matching with the respective patient's information.

The model training unit 208 performs data processing on the third data and the fourth data filtered by the filtering unit 206 in order to use them in training of the stroke volume calculation model.

The model training unit 208 may smooth the stroke volume value by performing Lowess smoothing processing on the stroke volume among the third data. This is because the stroke volume calculated by the APCO equipment is calculated by an equation and is output discretely. Therefore, this operation may be performed so as to match the output stroke volume with the stroke volume calculated based on the TDCO equipment, which is output as continuous and smoothed data.

The model training unit 208 may perform delay processing on the fourth data as much as a preset time. This is because the TDCO equipment outputs data with a slight delay when actually outputting the same. Therefore, this operation is intended to use the delay-averaged value in training so as to reflect it in the actual modeling. However, when there is no delay in the fourth data itself, delay averaging may be omitted.

The model training unit 208 trains the stroke volume calculation model, which is a deep learning model, by using the smooth-processed third data and patient's information matching with the third data (first data). The stroke volume calculation model calculates a stroke volume for a specific patient when inputting information such as an arterial blood pressure, gender, age, height, weight, etc. of the specific patient.

The model training unit 208 transfer learns the trained stroke volume calculation model, by using the delay-averaged fourth data and information of the specific patient matching with the fourth data (second data). Finally, this unit verifies accuracy of the stroke volume calculation model measured with a pulmonary artery catheter collected prospectively using the transfer-learned stroke volume calculation model.

The stroke volume calculation unit 210 calculates a stroke volume of a specific patient using the arterial blood pressure of the specific patient and patient's information input or received through the stroke volume calculation model that has been finally verified.

The transmission unit 212 transmits the stroke volume of the specific patient calculated through the stroke volume calculation model to an external monitor or an external server in real time.

However, the stroke volume calculation device 100 using AI may also include the monitor unit.

FIG. 2B is a block diagram schematically illustrating a filtering unit of the device for calculating stroke volume using AI according to an embodiment of the present invention.

The filtering unit 206 includes a data extraction unit 214 and a data verification unit 216.

The data extraction unit 214 observes the stroke volume values in the first data and the second data, and when the stroke volume waveform changes 1 mL or more, extracts the arterial blood pressure values from before a preset time to a first time point at the first time point. In addition, a stroke volume corresponding to the extracted arterial blood pressure value is extracted. The preset time is, for example, 20 seconds.

The data verification unit 216 performs data verification on the arterial blood pressure values extracted from the first data and the second data, respectively, and the stroke volumes corresponding to the extracted arterial blood pressure values. The data verification unit 216 determines whether the arterial blood pressure value among the sample data extracted from the first data or the sample data extracted from the second data is within a first preset range. The data verification unit 216 does not use a value in which the arterial blood pressure value among the extracted sample data is not within the first preset range in training of the stroke volume calculation model. The first preset range may be, for example, 25 or more and 250 or less.

In addition, the data verification unit 216 determines whether the stroke volume among the sample data extracted from the first data or the sample data extracted from the second data is within a second preset range. The data verification unit 216 does not use a value in which the stroke volume among the extracted sample data is not within the second preset range in training of the stroke volume calculation model. The second preset range may be, for example, 20 or more and 200 or less.

In addition, the data verification unit 216 also excludes data from the sample data, in which the calculated standard deviation per bit is zero (0), among the sample data extracted from the first data or sample data extracted from the second data.

The data verified by the data verification unit 216 as described above are referred to as third data or fourth data, respectively. That is, data verified after data extraction among the first data is referred to as third data, and data verified after data extraction among the second data is referred to as fourth data.

FIG. 2C is a block diagram schematically illustrating a model training unit of the device for calculating stroke volume using AI according to an embodiment of the present invention.

The model training unit 208 includes a data processing unit 218, a pre-training unit 220, a transfer learning unit 222, and a model verification unit 224.

The data processing unit 218 performs data processing on the third data and the fourth data filtered by the filtering unit 206 in order to use them in training of the stroke volume calculation model.

The data processing unit 218 may smooth the stroke volume value by performing smoothing processing on the stroke volume among the third data. This is because the stroke volume calculated by the APCO equipment is calculated by an equation and is output discretely. Therefore, this operation may be performed so as to match the output stroke volume with the stroke volume calculated based on the TDCO equipment, which is output as continuous and smoothed data.

The data processing unit 218 may perform delay processing on the fourth data as much as 2 minutes, for example. This is because the TDCO equipment outputs data with a slight delay when actually outputting the same. Therefore, this operation is intended to use the delay-averaged value in training so as to reflect it in the actual modeling. However, when there is no delay in the fourth data itself, delay averaging may be omitted.

The pre-training unit 220 trains the stroke volume calculation model, which is a deep learning model, by using the smooth-processed third data and patient's information matching with the third data (first data). The stroke volume calculation model calculates a stroke volume for a specific patient when inputting information such as an arterial blood pressure, gender, age, height, weight, etc. of the specific patient.

The transfer learning unit 222 transfer learns the trained stroke volume calculation model, by using the delay-averaged fourth data and information of the specific patient matching with the fourth data (second data).

The pre-training or transfer learning method is a well-known deep learning technique, and therefore will not be described in detail.

The model verification unit 224 verifies the transfer-learned stroke volume calculation model using the fourth data. Then, this unit determines whether an error range of the stroke volume calculated through the transfer-learned stroke volume calculation model and the fourth data corresponding thereto is within a preset range, for example, 10%. If the error range is out of the preset range, the transfer learning unit 222 recalculates the stroke volume calculation model.

Then, the stroke volume of a specific patient is calculated using the arterial blood pressure of the specific patient and patient's information input or received through the stroke volume calculation model that has been finally verified.

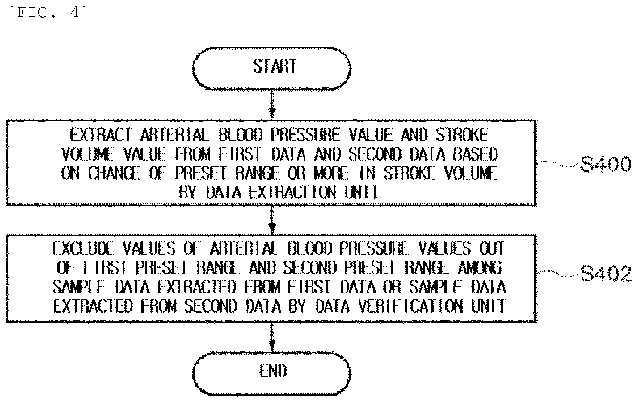

FIG. 4 is a flowchart illustrating a sequence of a method for calculating stroke volume using AI according to an embodiment of the present invention.

The filtering unit 206 observes stroke volume waveforms in the first data and the second data, thus to extract arterial blood pressure values from before a preset time to a first time point at the first time point where a change slope at which the stroke volume waveform changes at an arbitrary point in time is a preset slope value or more (S400). In addition, a stroke volume corresponding to the extracted arterial blood pressure value is extracted. The preset time is, for example, 20 seconds.

Next, the filtering unit 206 performs data verification on the arterial blood pressure values extracted from the first data and the second data, respectively, and the stroke volumes corresponding to the extracted arterial blood pressure values (S402). The filtering unit 206 determines whether the arterial blood pressure value among the sample data extracted from the first data or the sample data extracted from the second data is within a first preset range. The filtering unit 206 does not use a value in which the arterial blood pressure value among the extracted sample data is not within the first preset range in training of the stroke volume calculation model. The first preset range may be, for example, 25 or more and 250 or less. That is, when the arterial blood pressure value among the extracted sample data is less than 25 or more than 250, the filtering unit 206 excludes it from the sample data.

In addition, the filtering unit 206 determines whether the stroke volume among the sample data extracted from the first data or the sample data extracted from the second data is within a second preset range. The filtering unit 206 does not use a value in which the stroke volume among the extracted sample data is not within the second preset range in training of the stroke volume calculation model. The second preset range may be, for example, 20 or more and 200 or less. That is, when the stroke volume among the extracted sample data is less than 20 or more than 200, the filtering unit 206 excludes it from the sample data.

In addition, the filtering unit 206 also excludes data from the sample data, in which the calculated standard deviation per bit is zero (0), among the sample data extracted from the first data or sample data extracted from the second data.

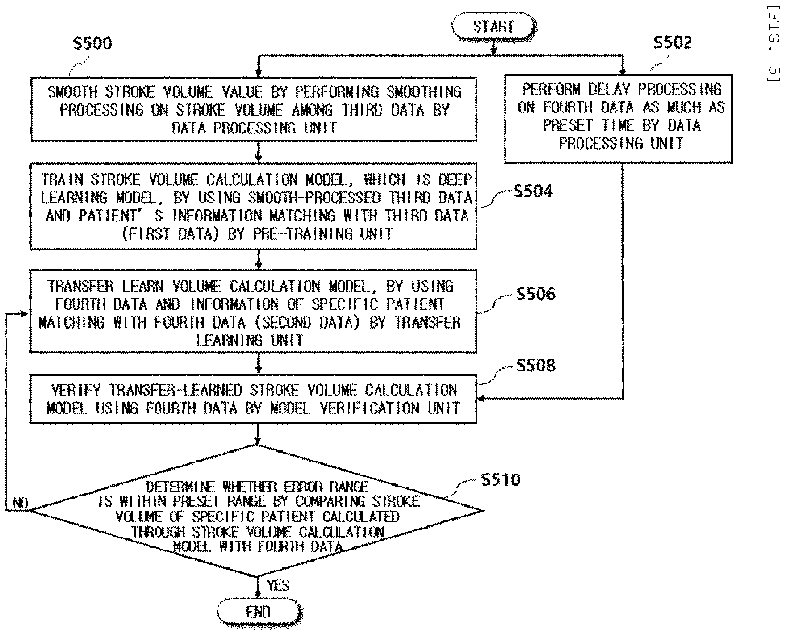

FIG. 5 is a flowchart illustrating a sequence of the method for calculating stroke volume using AI according to an embodiment of the present invention.

The data processing unit 218 performs data processing on the third data and the fourth data filtered by the filtering unit 206 in order to use them in training of the stroke volume calculation model.

The data processing unit 218 may smooth the stroke volume value by performing smoothing processing on the stroke volume among the third data (S500). This is because the stroke volume calculated by the APCO equipment is calculated by an equation and is output discretely. Therefore, this operation may be performed so as to match the output stroke volume with the stroke volume calculated based on the TDCO equipment, which is output as continuous and smoothed data.

The data processing unit 218 may perform delay processing on the fourth data as much as a preset time

(S502). This is because the TDCO equipment outputs data with a slight delay when actually outputting the same. Therefore, this operation is intended to use the delay-averaged value in training so as to reflect it in the actual modeling. However, when there is no delay in the fourth data itself, delay averaging may be omitted.

The pre-training unit 220 trains the stroke volume calculation model, which is a deep learning model, by using the Lowess smooth-processed third data and patient's information matching with the third data (first data) (S504). The stroke volume calculation model calculates a stroke volume for a specific patient when inputting information such as an arterial blood pressure, gender, age, height, weight, etc. of the specific patient.

The transfer learning unit 222 transfer learns the trained stroke volume calculation model, by using the delay-averaged fourth data and information of the specific patient matching with the fourth data (second data) (S506).

The model verification unit 224 verifies the transfer-learned stroke volume calculation model using the fourth data (S508). Then, this unit determines whether an error range of the stroke volume calculated through the transfer-learned stroke volume calculation model and the fourth data corresponding thereto is within a preset range, for example, 10%. If the error range is out of the preset range, the transfer learning unit 222 recalculates the stroke volume calculation model.

Then, this unit determines whether the error range is within the preset range by comparing the stroke volume of the specific patient calculated through the stroke volume calculation model with the fourth data (S510). If the error range is out of the preset range, the transfer learning unit 222 transfer relearns the stroke volume calculation model by using the fourth data.

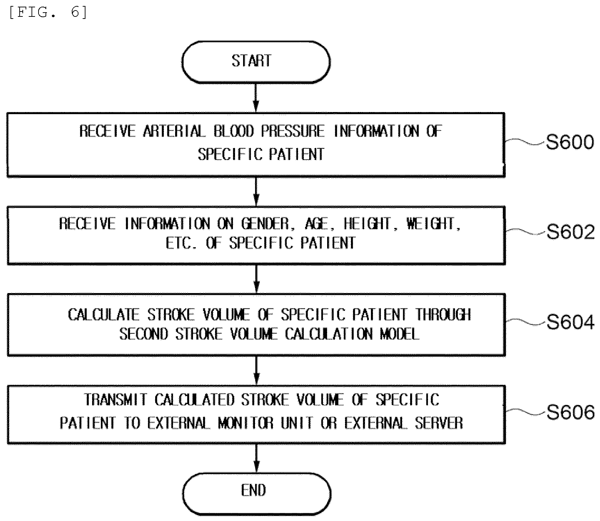

FIG. 6 is a flowchart illustrating a sequence of the method for calculating stroke volume using AI according to an embodiment of the present invention.

Arterial blood pressure information of a specific patient is input by the input unit 200 or received by the reception unit 202 (S600). In addition, information of a specific patient, for example, information on the gender, age, height, weight, etc. of the specific patient is input by the input unit 200 or received by the reception unit 202 (S602).

The stroke volume calculation unit 210 calculates a stroke volume of the specific patient using information such as the arterial blood pressure, gender, age, height, weight, etc. of the specific patient received through the finally calculated stroke volume calculation model (S604). However, the stroke volume calculation unit 210 may calculate the stroke volume of the specific patient using only the arterial blood pressure of the specific patient received through the finally calculated stroke volume calculation model.

The transmission unit 212 transmits the calculated stroke volume of the specific patient to an external monitor unit or an external server (5606). However, the stroke volume calculation device 100 using AI may include the monitor unit.

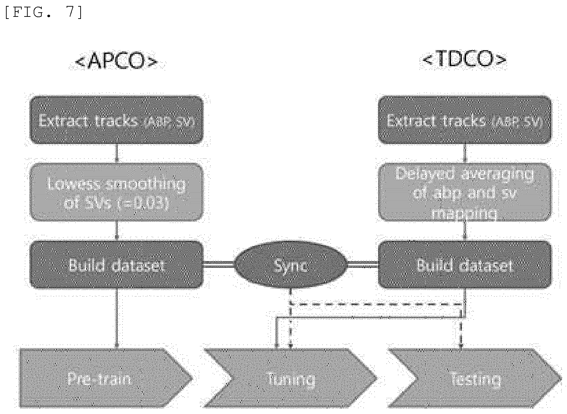

FIG. 7 is a diagram illustrating an example of a sequence in which a stroke volume is calculated using the stroke volume calculation model according to an embodiment of the present invention.

In the stroke volume calculation device 100 using AI, a first model is subjected to pre-training using large-scale APCO data. Thereafter, a tuning (transfer learning) process is performed on the data using relatively small TDCO data. In this case, in a process of acquiring TDCO data, time synchronization is performed on the data by shifting the processing time delay of the TDCO equipment (e.g., Vigilance II of Edward Lifesciences). The tuning process is performed using this data. A final test is also performed on the data extracted from the TDCO equipment and subjected to synchronization processing.

In the present specification, 'an embodiment' of the principles of the present invention and designation of various modifications of the expression denote that a specific feature, structure, and characteristic are included in at least one embodiment of the principle of the present invention. Therefore, the expression 'in an embodiment' and arbitrary other modification examples disclosed herein do not necessarily refer to the same embodiment.

All embodiments and conditional embodiments disclosed through the present specification are described with an intention to help the persons who have a common knowledge in the technical field to which the invention pertains to understand the principles and concepts of the present invention, and it will be understood by those skilled in the art that the present invention may be implemented in a modified form without departing from the essential characteristics of the present invention. Therefore, the embodiments described in this disclosure should not be construed to limit the technical idea of the present invention, but should be construed to illustrate the technical idea of the present invention. The scope of the present invention is defined in the appended claims.

## Claims

1. A device (100) for calculating stroke volume using AI comprising:
a filtering unit (206) configured to filter an arterial blood pressure value and a stroke volume which are in a preset range, among first data and second data comprising a plurality of arterial blood pressure values and a plurality of stroke volumes corresponding to the plurality of arterial blood pressure values;
a pre-training unit (220) configured to pre-train a first stroke volume calculation model which calculates a stroke volume based on the arterial blood pressure value, by using third data filtered from the first data;
a transfer learning unit (222) configured to transfer learn the first stroke volume calculation model, by using fourth data filtered from the second data, thus to generate a second stroke volume calculation model; and
a stroke volume calculation unit (210) configured to calculate a stroke volume corresponding to the input arterial blood pressure of a specific patient by using the second stroke volume calculation model,
**characterized in that** the filtering unit (206) comprises:
a data extraction unit configured to extract arterial blood pressure values and corresponding stroke volumes from a preset time before a first time point to the first time point, from the first data and the second data, wherein the first time point is a time point where the change slope of the stroke volume waveform reaches or exceeds a preset slope value.

2. The device (100) for calculating stroke volume using AI according to claim 1, further comprising a storage unit (204) configured to store the first data and the second data, and patient's information corresponding to the first data and the second data, respectively,
wherein, when training the first stroke volume calculation model by the pre-training unit (220), the patient's information corresponding to the first data is used together,
when generating the second stroke volume calculation model by the transfer learning unit (222), transfer learning is performed by using patient's information corresponding to the second data together, and
the patient's information includes at least one of gender, age, weight, and height of the patient.

3. The device (100) for calculating stroke volume using AI according to claim 1 or 2, wherein the filtering unit (206) further comprises:
a data verification unit (216) configured to extract a value within a first preset range from among the extracted arterial blood pressure values, and extract a value within a second preset range from among the extracted stroke volumes;
wherein data extracted from the first data is referred to as third data, and data extracted from the second data is referred to as fourth data.

4. The device (100) for calculating stroke volume using AI according to claim 3, wherein the preset time is 20 seconds, the first preset range is 20 or more and 250 or less, and the second preset range is 20 or more and 200 or less.

5. The device (100) for calculating stroke volume using AI according to claim 4, wherein the data verification unit (216) excludes a value, in which an average deviation per bit calculated from an arterial blood pressure waveform is zero (0), among the third data and the fourth data.

6. The device (100) for calculating stroke volume using AI according to claim 5,
further comprising a data processing unit configured to perform smoothing processing on the stroke volume among the third data, and delay the fourth data as much as a preset time,
wherein the first stroke volume calculation model is subjected to training using the data processed by the data processing unit to generate the second stroke volume calculation model.

7. The device (100) for calculating stroke volume using AI according to claim 6, further comprising a stroke volume calculation model verification unit configured to determine whether an error range of the stroke volume calculated through the second stroke volume calculation model and the fourth data is within a preset range to verify the second stroke volume calculation model,
wherein, if an error of the stroke volume calculated through the second stroke volume calculation model and the stroke volume of the fourth data corresponding thereto is out of a preset range, the transfer learning unit (222) relearns the second stroke volume calculation model.

8. The device (100) for calculating stroke volume using AI according to claim 1, wherein the first data is data calculated using arterial pressure-based cardiac output (APCO) equipment, and
the second data is data calculated using thermodilution-based cardiac output (TDCO) equipment.

9. A method for calculating stroke volume using AI which calculates a stroke volume from an arterial blood pressure of a specific patient by using a device (100) for calculating stroke volume using AI, the method comprising:
filtering an arterial blood pressure value and a stroke volume which are in a preset range, among first data and second data comprising a plurality of arterial blood pressure values and a plurality of stroke volumes corresponding to the plurality of arterial blood pressure values;
pre-training (S504) a first stroke volume calculation model which calculates a stroke volume based on the arterial blood pressure value, by using third data filtered from the first data;
transfer-learning (S506) the first stroke volume calculation model, by using fourth data filtered from the second data, thus to generate a second stroke volume calculation model; and
calculating (S604) a stroke volume corresponding to the input arterial blood pressure of a specific patient by using the second stroke volume calculation model,
**characterized in that** the filtering comprising:
extracting (S400) arterial blood pressure values and corresponding stroke volumes from a preset time before a preset time point to the first time point, from the first data and the second data,
wherein the first time point is a time point where the change slope of the stroke volume waveform reaches or exceeds a preset slope value.

10. The method according to claim 9, wherein, when training the first stroke volume calculation model, patient's information corresponding to the first data is used together,
when generating the second stroke volume calculation model, transfer learning is performed by using patient's information corresponding to the second data together, and
the patient's information includes at least one of gender, age, weight, and height of the patient.

11. The method according to claim 9 or 10, further comprising:
extracting (S402) a value within a first preset range from among the extracted arterial blood pressure values, and extracting a value within a second preset range from among the extracted stroke volumes;
wherein data extracted from the first data is referred to as third data, and data extracted from the second data is referred to as fourth data.

12. The method according to claim 11, wherein the preset time is 20 seconds, the first preset range is 20 or more and 250 or less, and the second preset range is 20 or more and 200 or less.

13. The method according to claim 12, wherein in the step of extracting as sample data,
excluding a value, in which an average deviation per bit calculated from an arterial blood pressure waveform is zero (0), among the third data and the fourth data.

14. The method according to claim 13, further comprising:
performing Lowess smoothing (S500) processing on the stroke volume among the third data, and delaying (S502) the fourth data as much as a preset time; and
wherein the first stroke volume calculation model is subjected to training using the data processed by the data processing unit to generate the second stroke volume calculation model.

15. The method according to claim 14, further comprising: determining whether an error range of the stroke volume calculated through the second stroke volume calculation model and the fourth data is within a preset range to verify the second stroke volume calculation model,
wherein, if an error of the stroke volume calculated through the second stroke volume calculation model and the stroke volume of the fourth data corresponding thereto is out of a preset range, relearning the second stroke volume calculation model.

## Patentansprüche

1. Vorrichtung (100) zum Berechnen des Schlagvolumens mit Hilfe von KI, umfassend:
eine Filtereinheit (206), die so konfiguriert ist, dass sie aus ersten Daten und zweiten Daten, die eine Vielzahl von arteriellen Blutdruckwerten und eine Vielzahl von Schlagvolumina, die der Vielzahl von arteriellen Blutdruckwerten entsprechen, umfassen, einen arteriellen Blutdruckwert und ein Schlagvolumen, die sich in einem voreingestellten Bereich befinden, herausfiltern kann;
eine Vortrainingseinheit (220), die so konfiguriert ist, dass sie ein erstes Schlagvolumen-Berechnungsmodell, das mit Hilfe von dritten Daten, die aus den ersten Daten herausgefiltert sind, ein Schlagvolumen auf der Basis des arteriellen Blutdruckwerts berechnet, vortrainieren kann;
eine Transferlerneinheit (222), die so konfiguriert ist, dass sie das erste Schlagvolumen-Berechnungsmodell einem Transferlernen unterziehen kann, indem sie aus den zweiten Daten herausgefilterte vierte Daten verwendet, wodurch ein zweites Schlagvolumen-Berechnungsmodell entsteht; und
eine Schlagvolumen-Berechnungseinheit (210), die so konfiguriert ist, dass sie ein Schlagvolumen, das dem eingegebenen arteriellen Blutdruck eines speziellen Patienten entspricht, berechnen kann, indem sie das zweite Schlagvolumen-Berechnungsmodell verwendet,
**dadurch gekennzeichnet, dass** die Filtereinheit (206) Folgendes umfasst:
eine Datenextraktionseinheit, die so konfiguriert ist, dass sie arterielle Blutdruckwerte und entsprechende Schlagvolumina von einem voreingestellten Zeitpunkt vor einem ersten Zeitpunkt bis zu dem ersten Zeitpunkt aus den ersten Daten und den zweiten Daten extrahieren kann, wobei der erste Zeitpunkt ein Zeitpunkt ist, an dem die Änderungssteigung der Schlagvolumen-Wellenform einen voreingestellten Steigungswert erreicht oder überschreitet.

2. Vorrichtung (100) zum Berechnen des Schlagvolumens mit Hilfe von KI gemäß Anspruch 1, weiterhin umfassend eine Speichereinheit (204), die so konfiguriert ist, dass sie die ersten Daten und die zweiten Daten sowie die den ersten Daten bzw. den zweiten Daten entsprechenden Patienteninformationen speichern kann,
wobei dann, wenn das erste Schlagvolumen-Berechnungsmodell von der Vortrainingseinheit (220) trainiert wird, die Informationen des Patienten, die den ersten Daten entsprechen, zusammen verwendet werden,
dann, wenn das zweite Schlagvolumen-Berechnungsmodell von der Transferlerneinheit (222) erzeugt wird, das Transferlernen durchgeführt wird, indem man Informationen des Patienten, die den zweiten Daten entsprechen, zusammen verwendet, und
die Informationen des Patienten wenigstens eine aus dem Geschlecht, Alter, Gewicht und der Größe des Patienten umfassen.

3. Vorrichtung (100) zum Berechnen des Schlagvolumens mit Hilfe von KI gemäß Anspruch 1 oder 2, wobei die Filtereinheit (206) weiterhin Folgendes umfasst:
eine Datenüberprüfungseinheit (216), die so konfiguriert ist, dass sie einen Wert innerhalb eines ersten voreingestellten Bereichs aus den extrahierten arteriellen Blutdruckwerten extrahiert und einen Wert innerhalb eines zweiten voreingestellten Bereichs aus den extrahierten Schlagvolumina extrahiert;
wobei aus den ersten Daten extrahierte Daten als "dritte Daten" bezeichnet werden und aus den zweiten Daten extrahierte Daten als "vierte Daten" bezeichnet werden.

4. Vorrichtung (100) zum Berechnen des Schlagvolumens mit Hilfe von KI gemäß Anspruch 3, wobei die voreingestellte Zeit 20 Sekunden beträgt, der erste voreingestellte Bereich 20 oder mehr und 250 oder weniger umfasst und der zweite voreingestellte Bereich 20 oder mehr und 200 oder weniger umfasst.

5. Vorrichtung (100) zum Berechnen des Schlagvolumens mit Hilfe von KI gemäß Anspruch 4, wobei die Datenüberprüfungseinheit (216) einen Wert, bei dem die aus einer Wellenform des arteriellen Blutdrucks berechnete mittlere Abweichung pro Bit Null (0) beträgt, aus den dritten Daten und den vierten Daten ausschließt.

6. Vorrichtung (100) zum Berechnen des Schlagvolumens mit Hilfe von KI gemäß Anspruch 5,
weiterhin umfassend eine Datenverarbeitungseinheit, die so konfiguriert ist, dass sie unter den dritten Daten eine Glättungsverarbeitung mit dem Schlagvolumen durchführen und die vierten Daten um eine voreingestellte Zeit verzögern kann,
wobei das erste Schlagvolumen-Berechnungsmodell einem Training unter Verwendung der von der Datenverarbeitungseinheit verarbeiteten Daten unterzogen wird, um das zweite Schlagvolumen-Berechnungsmodell zu erzeugen.

7. Vorrichtung (100) zum Berechnen des Schlagvolumens mit Hilfe von KI gemäß Anspruch 6, weiterhin umfassend eine Schlagvolumen-Berechnungsmodell-Überprüfungseinheit, die so konfiguriert ist, dass sie bestimmen kann, ob ein Fehlerbereich des durch das zweite Schlagvolumen-Berechnungsmodell und die vierten Daten berechneten Schlagvolumens innerhalb eines voreingestellten Bereichs liegt, um das zweite Schlagvolumen-Berechnungsmodell zu überprüfen,
wobei dann, wenn ein Fehler des durch das zweite Schlagvolumen-Berechnungsmodell berechneten Schlagvolumens und des Schlagvolumens der entsprechenden vierten Daten außerhalb eines voreingestellten Bereichs liegt, die Transferlerneinheit (222) das zweite Schlagvolumen-Berechnungsmodell einem erneuten Lernen unterzieht.

8. Vorrichtung (100) zum Berechnen des Schlagvolumens mit Hilfe von KI gemäß Anspruch 1, wobei es sich bei den ersten Daten um Daten handelt, die mit einem Gerät zur Messung des arteriellen Drucks auf der Grundlage des Herzzeitvolumens (APCO) berechnet wurden, und
es sich bei den zweiten Daten um Daten handelt, die mit einem Gerät zur Messung des Herzzeitvolumens auf Thermodilutionsbasis (TDCO) berechnet wurden.

9. Verfahren zum Berechnen des Schlagvolumens mit Hilfe von KI, das ein Schlagvolumen aus einem arteriellen Blutdruck eines speziellen Patienten berechnet, indem es eine Vorrichtung (100) zum Berechnen des Schlagvolumens mit Hilfe von KI verwendet, wobei das Verfahren umfasst:
das Herausfiltern eines arteriellen Blutdruckwerts und eines Schlagvolumens, die sich in einem voreingestellten Bereich befinden, aus ersten Daten und zweiten Daten, die eine Vielzahl von arteriellen Blutdruckwerten und eine Vielzahl von Schlagvolumina, die der Vielzahl von arteriellen Blutdruckwerten entsprechen, umfassen;
das Vortrainieren (S504) eines ersten Schlagvolumen-Berechnungsmodells, das mit Hilfe von dritten Daten, die aus den ersten Daten herausgefiltert sind, ein Schlagvolumen auf der Basis des arteriellen Blutdruckwerts berechnet;
das Durchführen eines Transferlernens (S506) mit dem ersten Schlagvolumen-Berechnungsmodell, indem man aus den zweiten Daten herausgefilterte vierte Daten verwendet, wodurch ein zweites Schlagvolumen-Berechnungsmodell entsteht; und
das Berechnen (S604) eines Schlagvolumens, das dem eingegebenen arteriellen Blutdruck eines speziellen Patienten entspricht, indem man das zweite Schlagvolumen-Berechnungsmodell verwendet,
**dadurch gekennzeichnet, dass** das Herausfiltern Folgendes umfasst:
das Extrahieren (S400) von arteriellen Blutdruckwerten und entsprechenden Schlagvolumina von einem voreingestellten Zeitpunkt vor einem ersten Zeitpunkt bis zu dem ersten Zeitpunkt aus den ersten Daten und den zweiten Daten,
wobei der erste Zeitpunkt ein Zeitpunkt ist, an dem die Änderungssteigung der Schlagvolumen-Wellenform einen voreingestellten Steigungswert erreicht oder überschreitet.

10. Verfahren gemäß Anspruch 9, wobei beim Trainieren des ersten Schlagvolumen-Berechnungsmodells die Informationen des Patienten, die den ersten Daten entsprechen, zusammen verwendet werden,
beim Erzeugen des zweiten Schlagvolumen-Berechnungsmodells das Transferlernen durchgeführt wird, indem man Informationen des Patienten, die den zweiten Daten entsprechen, zusammen verwendet und
die Informationen des Patienten wenigstens eine aus dem Geschlecht, Alter, Gewicht und der Größe des Patienten umfassen.

11. Verfahren gemäß Anspruch 9 oder 10, weiterhin umfassend:
das Extrahieren (S402) eines Werts innerhalb eines ersten voreingestellten Bereichs aus den extrahierten arteriellen Blutdruckwerten und das Extrahieren eines Werts innerhalb eines zweiten voreingestellten Bereichs aus den extrahierten Schlagvolumina;
wobei aus den ersten Daten extrahierte Daten als "dritte Daten" bezeichnet werden und aus den zweiten Daten extrahierte Daten als "vierte Daten" bezeichnet werden.

12. Verfahren gemäß Anspruch 11, wobei die voreingestellte Zeit 20 Sekunden beträgt, der erste voreingestellte Bereich 20 oder mehr und 250 oder weniger umfasst und der zweite voreingestellte Bereich 20 oder mehr und 200 oder weniger umfasst.

13. Verfahren gemäß Anspruch 12, wobei in dem Schritt des Extrahierens von Probendaten
ein Wert, bei dem die aus einer Wellenform des arteriellen Blutdrucks berechnete mittlere Abweichung pro Bit Null (0) beträgt, aus den dritten Daten und den vierten Daten ausgeschlossen wird.

14. Verfahren gemäß Anspruch 13, weiterhin umfassend:
das Durchführen einer Lowess-Glättungsverarbeitung (S500) mit dem Schlagvolumen unter den dritten Daten und das Verzögern (S502) der vierten Daten um eine voreingestellte Zeit;
wobei das erste Schlagvolumen-Berechnungsmodell einem Training unter Verwendung der von der Datenverarbeitungseinheit verarbeiteten Daten unterzogen wird, um das zweite Schlagvolumen-Berechnungsmodell zu erzeugen.

15. Verfahren gemäß Anspruch 14, weiterhin umfassend: das Bestimmen, ob ein Fehlerbereich des durch das zweite Schlagvolumen-Berechnungsmodell und die vierten Daten berechneten Schlagvolumens innerhalb eines voreingestellten Bereichs liegt, um das zweite Schlagvolumen-Berechnungsmodell zu überprüfen,
wobei dann, wenn ein Fehler des durch das zweite Schlagvolumen-Berechnungsmodell berechneten Schlagvolumens und des Schlagvolumens der entsprechenden vierten Daten außerhalb eines voreingestellten Bereichs liegt, das zweite Schlagvolumen-Berechnungsmodell einem erneuten Lernen unterzogen wird.

## Revendications

1. Dispositif (100) de calcul du volume systolique à l'aide de l'IA, comprenant :
une unité de filtrage (206) configurée pour filtrer une valeur de pression artérielle et un volume systolique qui sont dans une plage prédéfinie, parmi des premières données et des deuxièmes données comprenant une pluralité de valeurs de pression artérielle et une pluralité de volumes systoliques correspondant à la pluralité de valeurs de pression artérielle ;
une unité de pré-formation (220) configurée pour pré-former un premier modèle de calcul de volume systolique qui calcule un volume systolique sur la base de la valeur de la pression artérielle, en utilisant des troisièmes données filtrées à partir des premières données ;
une unité d'apprentissage par transfert (222) configurée pour transférer l'apprentissage du premier modèle de calcul de volume systolique, en utilisant des quatrièmes données filtrées à partir des deuxièmes données, afin de générer ainsi un deuxième modèle de calcul de volume systolique ; et
une unité de calcul de volume systolique (210) configurée pour calculer un volume systolique correspondant à la pression artérielle d'entrée d'un patient spécifique en utilisant le deuxième modèle de calcul de volume systolique,
**caractérisé en ce que** l'unité de filtrage (206) comprend :
une unité d'extraction de données configurée pour extraire des valeurs de pression artérielle et des volumes systoliques correspondants à partir d'un temps prédéfini avant un premier point temporel jusqu'au premier point temporel, à partir des premières données et des deuxièmes données, le premier point temporel étant un point temporel où la pente de changement de la forme d'onde du volume systolique atteint ou dépasse une valeur de pente prédéfinie.

2. Dispositif (100) de calcul du volume systolique à l'aide de l'IA selon la revendication 1, comprenant en outre : une unité de stockage (204) configurée pour stocker les premières données et les deuxièmes données, et les informations du patient correspondant aux premières données et aux deuxièmes données, respectivement,
dans lequel, lors de la formation du premier modèle de calcul du volume systolique par l'unité de pré-formation (220), les informations du patient correspondant aux premières données sont utilisées ensemble,
lors de la génération du deuxième modèle de calcul du volume systolique par l'unité d'apprentissage de transfert (222), l'apprentissage de transfert est effectué en utilisant les informations du patient correspondant aux deuxièmes données ensemble, et
les informations du patient comprennent au moins le sexe, l'âge, le poids et la taille du patient.

3. Dispositif (100) de calcul du volume systolique à l'aide de l'IA selon la revendication 1 ou 2, dans lequel l'unité de filtrage (206) comprend en outre
une unité de vérification de données (216) configurée pour extraire une valeur dans une première plage prédéfinie parmi les valeurs de pression artérielle extraites, et extraire une valeur dans une deuxième plage prédéfinie parmi les volumes systoliques extraits ;
dans lequel les données extraites des premières données sont appelées troisièmes données, et les données extraites des deuxièmes données sont appelées quatrièmes données.

4. Dispositif (100) de calcul du volume systolique à l'aide de l'IA selon la revendication 3, dans lequel le temps prédéfini est de 20 secondes, la première plage prédéfinie est de 20 ou plus et de 250 ou moins, et la deuxième plage prédéfinie est de 20 ou plus et de 200 ou moins.

5. Dispositif (100) de calcul du volume systolique à l'aide de l'IA selon la revendication 4, dans lequel l'unité de vérification de données (216) exclut une valeur, dans laquelle un écart moyen par bit calculé à partir d'une forme d'onde de pression artérielle est nul (0), parmi les troisièmes données et les quatrièmes données.

6. Dispositif (100) de calcul du volume systolique à l'aide de l'IA selon la revendication 5,
comprenant en outre une unité de traitement de données configurée pour effectuer un traitement de lissage sur le volume systolique parmi les troisièmes données, et retarder les quatrièmes données jusqu'à un temps prédéfini,
dans lequel le premier modèle de calcul de volume systolique est soumis à une formation à l'aide des données traitées par l'unité de traitement de données pour générer le deuxième modèle de calcul de volume systolique.

7. Dispositif (100) de calcul du volume systolique à l'aide de l'IA selon la revendication 6, comprenant en outre : une unité de vérification de modèle de calcul de volume systolique configurée pour déterminer si une plage d'erreur du volume systolique calculé via le deuxième modèle de calcul de volume systolique et les quatrièmes données se situe dans une plage prédéfinie pour vérifier le deuxième modèle de calcul de volume systolique,
dans lequel, si une erreur du volume systolique calculé par le biais du deuxième modèle de calcul de volume systolique et du volume systolique des quatrièmes données correspondant à celui-ci est hors d'une plage prédéfinie, l'unité d'apprentissage de transfert (222) réapprend le deuxième modèle de calcul de volume systolique.

8. Dispositif (100) de calcul du volume systolique à l'aide de l'IA selon la revendication 1, dans lequel les premières données sont des données calculées à l'aide d'un équipement de débit cardiaque basé sur la pression artérielle (APCO), et
les deuxièmes données sont des données calculées à l'aide d'un équipement de débit cardiaque basé sur la thermodilution (TDCO).

9. Méthode de calcul du volume systolique utilisant l'IA qui calcule un volume systolique à partir de la pression artérielle d'un patient spécifique en utilisant un dispositif (100) de calcul du volume systolique à l'aide de l'IA, ladite méthode comprenant les étapes consistant à :
filtrer une valeur de pression artérielle et un volume systolique qui sont dans une plage prédéfinie, parmi des premières données et des deuxièmes données comprenant une pluralité de valeurs de pression artérielle et une pluralité de volumes systoliques correspondant à la pluralité de valeurs de pression artérielle ;
pré-former (S504) un premier modèle de calcul de volume systolique qui calcule un volume systolique sur la base de la valeur de la pression artérielle, en utilisant des troisièmes données filtrées à partir des premières données ;
soumettre le premier modèle de calcul de volume systolique à un apprentissage par transfert (S506) en utilisant des quatrièmes données filtrées à partir des deuxièmes données, afin de générer ainsi un deuxième modèle de calcul de volume systolique ; et
calculer (S604) un volume systolique correspondant à la pression artérielle d'entrée d'un patient spécifique en utilisant le deuxième modèle de calcul de volume systolique,
**caractérisée en ce que** l'étape de filtrage comprend l'étape consistant à :
extraire (S400) des valeurs de pression artérielle et des volumes systoliques correspondants à partir d'un temps prédéfini avant un premier point temporel jusqu'au premier point temporel, à partir des premières données et des deuxièmes données,
dans laquelle le premier point temporel est un point temporel où la pente de changement de la forme d'onde du volume systolique atteint ou dépasse une valeur de pente prédéfinie.

10. Méthode selon la revendication 9, dans laquelle lors de la formation du premier modèle de calcul du volume systolique, les informations du patient correspondant aux premières données sont utilisées ensemble,
lors de la génération du deuxième modèle de calcul du volume systolique, l'apprentissage de transfert est effectué en utilisant les informations du patient correspondant aux deuxièmes données ensemble, et
les informations du patient comprennent au moins le sexe, l'âge, le poids et la taille du patient.

11. Méthode selon la revendication 9 ou 10, comprenant en outre l'étape consistant à :
extraire (S402) une valeur dans une première plage prédéfinie parmi les valeurs de pression artérielle extraites, et extraire une valeur dans une deuxième plage prédéfinie parmi les volumes systoliques extraits ;
dans lequel les données extraites des premières données sont appelées troisièmes données, et les données extraites des deuxièmes données sont appelées quatrièmes données.

12. Méthode selon la revendication 11, dans laquelle le temps prédéfini est de 20 secondes, la première plage prédéfinie est de 20 ou plus et de 250 ou moins, et la deuxième plage prédéfinie est de 20 ou plus et de 200 ou moins.

13. Méthode selon la revendication 12, dans laquelle dans l'étape d'extraction des données d'échantillon,
une valeur est exclue, dans laquelle un écart moyen par bit calculé à partir d'une forme d'onde de pression artérielle est nul (0), parmi les troisièmes données et les quatrièmes données.

14. Méthode selon la revendication 13, comprenant en outre l'étape consistant à
effectuer un traitement de lissage Lowess (S500) sur le volume systolique parmi les troisièmes données, et retarder (S502) les quatrièmes données jusqu'à un temps prédéfini ; et
dans lequel le premier modèle de calcul de volume systolique est soumis à une formation à l'aide des données traitées par l'unité de traitement de données pour générer le deuxième modèle de calcul de volume systolique.

15. Méthode selon la revendication 14, comprenant en outre l'étape consistant à déterminer si une plage d'erreur du volume systolique calculé via le deuxième modèle de calcul de volume systolique et les quatrièmes données se situe dans une plage prédéfinie pour vérifier le deuxième modèle de calcul de volume systolique,
dans lequel, si une erreur du volume systolique calculé par le biais du deuxième modèle de calcul de volume systolique et du volume systolique des quatrièmes données correspondant à celui-ci est hors d'une plage prédéfinie, le deuxième modèle de calcul de volume systolique est réappris.
